# EUROPEAN PATENT SPECIFICATION

(11) **EP 2 882 728 B1**
(45) Date of publication and mention of the grant of the patent: **10.08.2016**
(21) Application number: 13748028.1
(22) Date of filing: 12.08.2013
(51) Int. Cl.: C07D 311/72

(54) **TOCOPHEROLS WITH REDUCED CONTENT OF DEHYDROTOCOPHEROL**
TOCOPHEROLE MIT REDUZIERTEM ANTEIL AN DEHYDROTOCOPHEROL
TOCOPHÉROLS À TENEUR RÉDUITE DE DÉHYDROTOCOPHÉROL

(30) Priority: 10.08.2012 EP 12180081
(43) Date of publication of application: 17.06.2015
(73) Proprietor: DSM IP Assets B.V., 6411 TE Heerlen (NL)
(72) Inventor: WILDERMANN, Angela, CH-4002 Basel (CH); EBNER, Niklas, CH-4002 Basel (CH); STAEUBLE, Viktor, CH-4002 Basel (CH); WEYLAND, Andreas, CH-4002 Basel (CH); DOORNENBAL, Otto, CH-4002 Basel (CH)
(74) Representative: Dux, Roland
(86) International application number: PCT/EP2013/066814
(87) International publication number: WO 2014/023845

(56) References cited:
- WO-A1-91/17985
- FR-A- 962 797
- ROSSI M ET AL: "The effect of bleaching and physical refining on color and minor components of palm oil", JOURNAL OF THE AMERICAN OIL CHEMISTS' SOCIETY, vol. 78, no. 10, October 2001 (2001-10), pages 1051-1055, XP002689679, ISSN: 0003-021X
- KEITO BOKI: "BLEACHING OF ALKALI-REFINED VEGETABLE OILS WITH CLAY MINERALS", JOURNAL OF THE AMERICAN OIL CHEMISTS' SOCIETY, SPRINGER, DE, vol. 69, no. 3, 1 March 1992 (1992-03-01), pages 232-236, XP000245388, ISSN: 0003-021X, DOI: 10.1007/BF02635892
- PROKSA B ET AL: "ISOLATION AND IDENTIFICATION OF SOME DEGRADATION PRODUCTS OF TOCOPHEROL AND ITS ACETATE", DIE PHARMAZIE, GOVI VERLAG PHARMAZEUTISCHER VERLAG GMBH, ESCHBORN, DE, vol. 39, no. 4, 1 January 1984 (1984-01-01), page 279, XP001204892, ISSN: 0031-7144
- Elisabeth Schwab: "Bleicherden", RÖMPP Online, Version 3.29 , August 2005 (2005-08), XP002689680, Retrieved from the Internet: URL:http://www.roempp.com/prod/?ID=RD-02-0 1912 [retrieved on 2012-12-21]
- SABA NAZ ET AL: "Changes of Total Tocopherol and Tocopherol Species During Sunflower Oil Processing", JOURNAL OF THE AMERICAN OIL CHEMISTS' SOCIETY, vol. 88, no. 1, 1 January 2011 (2011-01-01), pages 127-132, XP055048035, ISSN: 0003-021X, DOI: 10.1007/s11746-010-1652-4 cited in the application

## Description

### Technical Field

The invention relates to the field of preparation and use of tocopherols and protected tocopherols.

### Background of the invention

Tocopherols are important substances in the field of food and feed ingredients. It is already known for a long time that tocopherols are part of vitamin E and show an important bioactivity. In order to increase the stability of tocopherols the phenolic hydroxyl group can be protected, particularly as acetate. The protected tocopherol can be easily produced from the corresponding tocopherols and deprotected again easily into the tocopherol on demand, for example in a body of an animal or a human being. It is known that tocopherols and protected tocopherols comprise small amount of impurities. One important impurity is 3,4-dehydrotocopherol, respectively the protected form thereof. 3,4-dehydrotocopherols was also be found to occur naturally in roots of various *Stemona* species as disclosed in B. Brem et al., Phytochemistry 65 (2004) 2719-2729, which also discloses that the antioxidation behaviors of the 3,4-dehydrotocopherols are is comparable to the tocopherols. Despite this finding, it is desired to lower the level of impurity respectively even to eliminate this impurity completely.

Vegetable oils and fats have often undesired strong odors and colors and, hence, refinement is necessary to have an acceptable quality for the consumption by animals or humans. This refinement involves different purification steps of which bleaching is the most prominent step. It is known since a long time that bleaching earths are efficient in the refinement of edible oils and fats. Bleaching earths are also known as bleaching clays. It was found that for certain purposes, bleaching earths may be modified by the treatment with strong acids and that those acid-treated bleaching earths, which are also known as acid activated bleaching earths, exhibit a better performance in the bleaching. However, it is known, for example from J. Am. Oil Chem. Soc (2011) 88: 127-132, that the level of tocopherols being present in the oil to be refined is remarkably reduced by the bleaching step and that the adsorption of the tocopherols on the bleaching clays is regarded as reason for this. Therefore, often tocopherols are added to edible oils and fats to increase the level of tocopherols again in order to stabilize those oils against further oxidative deterioration such as disclosed in US 4,101,673.

FR 962 797 discloses that tocopherols can be obtained from different vegetable oils. It further discloses a method of purifying and/or separation of tocopherols from such sources by means of an adsorbent being clay, kaolin or silica gel. In this method first the tocopherol is adsorbed from a highly diluted solution in a non-polar solvent onto the adsorbent, preferably in a column, and, hence, is separated from the rest of the sourcing composition. In a second step the adsorbed tocopherol is then extracted from the adsorbent by percolating by means of another solvent being more polar. By using different solvents alpha and gamma tocopherols can be separated from each other and isolated from a mixture or concentrate as they result from natural vegetable oil sources. However, this purification is very expensive as it requires a large amount of adsorbent as well as of different solvents and is not suitable for being used for large quantities and highly concentrated forms of tocopherols as they result from chemical synthesis.

The need for tocopherol on the world's market is extremely large, and, therefore, the major part of commercially available tocopherols is synthesized from petrochemicals and is not originating from bio-sources. These syntheses yield tocopherol in neat form or in very highly concentrated solutions having 3,4-dehydrotocopherol as impurity.

### Summary of the invention

The problem to be solved by the present invention is to reduce the level of the impurity 3,4-dehydrotocopherol in tocopherol, or of the impurity protected 3,4-dehydrotocopherol in protected tocopherol, respectively, which exists in highly concentrated forms of tocopherols or protected tocopherols, respectively..

Surprisingly, it has been found that the method according to claim 1 is able to solve this problem. Particularly contacting the tocopherol or the protected tocopherol, respectively, with acid activated bleaching earth leads to a remarkable reduction of 3,4-dehydrotocopherol in tocopherol, or protected 3,4-dehydrotocopherol in protected tocopherol, respectively.

It is particularly surprising that exactly acid activated bleaching earths are suitable for this purpose in consideration of the fact that it is known that such adsorbing material exactly adsorbs in preference the product to be treated, i.e. the tocopherols.

The method is very efficient and easy and allows producing tocopherols or protected tocopherols (e.g. tocopheryl acetates) in high quality, thus, this method is very attractive to producers of vitamin E and to the market. It was particularly found that the amount of 3,4-dehydrotocopherol or of the protected form thereof can be reduced by more that 20% by weight, preferably by more than 30 % by weight, and even more preferably by more than 50 % by weight.

Further aspects of the invention are subject of further independent claims. Particularly preferred embodiments are subject of dependent claims.

### Detailed description of the invention

The present invention relates in a first aspect to a method of preparing a tocopherol or a protected tocopherol of formula (I) with a reduced content of dehydrotocopherol or protected dehydrotocopherol of formula (II) comprising the step of contacting tocopherol or protected tocopherol of formula (I), in the form of neat tocopherol or protected tocopherol of formula (I) or in the form of a solution comprising more than 60 % by weight of tocopherol or protected tocopherol of formula (I), with an acid activated bleaching earth 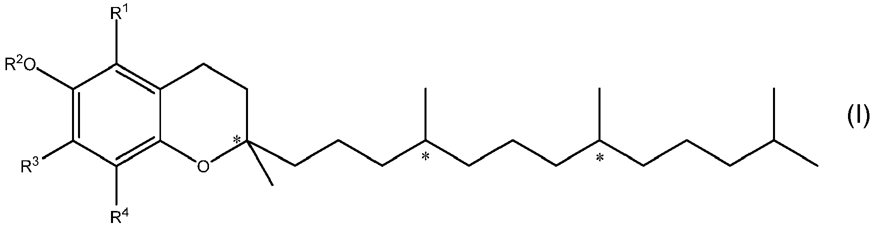 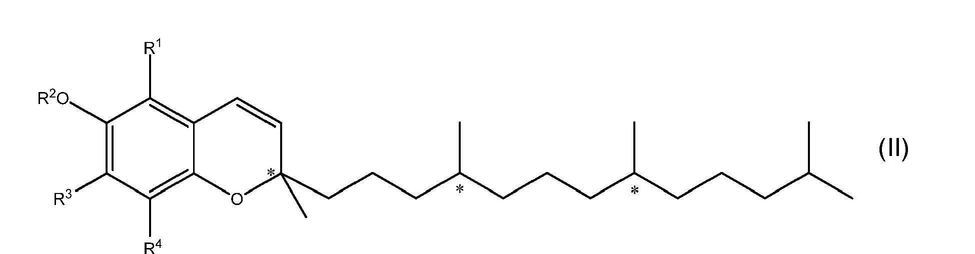
wherein R¹, R³ and R⁴ are independently from each other hydrogen or methyl groups;
R² represents hydrogen or a phenol protection group;
and wherein each * represents an individual chiral centre.

The term "independently from each other" in this document means, in the context of substituents, moieties, or groups, that identically designated substituents, moieties, or groups can occur simultaneously with a different meaning in the same molecule.

A "C_{x-y}-alkyl" or "C_{x-y}-acyl" group is an alkyl or an acyl group, respectively, comprising x to y carbon atoms, i.e., for example, a C₁₋₃-alkyl group is an alkyl group comprising 1 to 3 carbon atoms. The alkyl group or the acyl group can be linear or branched.

The term "hydrogen" means in the present document H and not H₂.

R² represents hydrogen or a phenol protection group. A phenol protection group is a group which protects the phenolic group (OH in formula (I)) and can be deprotected easily, i.e. by state-of-the-art methods, to the phenolic group again.

When the term "tocopherol of formula (I)" is used in this document the compound of formula (I) having hydrogen as R² is meant.

When the term "protected tocopherol of formula (I)" is used in this document the compound of formula (I) having a protection group as R² is meant.

When the term "tocopherol or protected tocopherol of formula (I)" is used in this document the compound of formula (I) having a hydrogen or a protection group as R² is meant.

The dehydrotocopherol or protected dehydrotocopherol of formula (II) carries the same groups R¹, R², R³ and R⁴ as the tocopherol or protected tocopherol of formula (I). For example, if formula (I) has the groups R¹ = R⁴ = CH₃, R³ = R² = H, then also formula (II) has the groups R¹ = R⁴ = CH₃, R³ = R² = H.

The chirality at the individual chiral centres indicated by * of the dehydrotocopherol or protected dehydrotocopherol of formula (II) correspond accordingly also to the chirality at the individual corresponding chiral centres of the tocopherol or protected tocopherol of formula (I).

The phenol protection group forms with the rest of the molecule a chemical functionality which is selected from the group consisting of ester, ether or acetal. The protection group can be easily removed by standard methods known to the person skilled in the art.

In case where the phenol protection group forms with the rest of the molecule an ether, the substituent R² is particularly a linear or branched C₁₋₁₀-alkyl or cycloalkyl or aralkyl group. Preferably the substituent R² is a benzyl group or a substituted benzyl group, particularly preferred a benzyl group.

In case where the phenol protection group forms with the rest of the molecule an ester, the ester is an ester of an organic or inorganic acid.

If the ester is an ester of an organic acid, the organic acid can be a monocarboxylic acid or a polycarboxylic acid, i.e. an acid having two or more COOH-groups. Polycarboxylic acids are preferably malonic acid, succinic acid, glutaric acid, adipic acid, maleic acid or fumaric acid.

Preferably the organic acid is a monocarboxylic acid.

Hence, the substituent R² is preferably an acyl group. The acyl group is particularly a C₁₋₇-acyl, preferably acetyl, propionyl or benzoyl group, or a substituted benzoyl group.

If the ester is an ester of an inorganic acid, the inorganic acid is preferably nitric acid or a polyprotic acid, i.e. an acid able to donate more than one proton per acid molecule, particularly selected from the group consisting of phosphoric acid, pyrophosphoric acid (=diphosphoric acid), phosphorous acid (=phosphonic acid), sulphuric acid and sulphurous acid.

In case where the phenol protection group forms with the rest of the molecule an acetal, the substituent R² is preferably 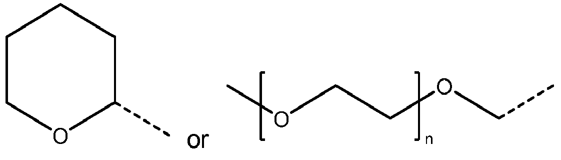 with n=0 or 1.

Hence, the acetals formed are preferably methoxymethyl ether (MOM-ether), β-methoxyethoxymethyl ether (MEM-ether) or tetrahydropyranyl ether (THP-ether). The protection group can easily be removed by acid.

In the present document any dotted line represents the bond by which a substituent is bound to the rest of a molecule.

The tocopherol of formula (I) is reacted with a protecting agent to yield a protected tocopherol of formula (I).

The protecting agents leading to the corresponding phenol protection groups are known to the person skilled in the art, as well as the chemical process and conditions for this reaction. If, for example, the phenol protection group forms with the rest of the molecule an ester, the suitable protecting agent is for example an acid, an anhydride or an acyl halide.

In the case that an ester is formed by the reaction of tocopherol of formula (I) with a protecting agent, and that said ester is an ester of an organic polycarboxylic acid or an inorganic polyprotic acid, not necessarily all acid groups are esterified to qualify as protected tocopherols of formula (I) in the sense of this document. Preferable esters of inorganic polyprotic acids, corresponding to protected tocopherols of formula (I), are tocopheryl phosphates and ditocopheryl phosphates, particularly α-tocopheryl phosphate and α-ditocopheryl phosphate.

It is preferred that the protection group is a benzoyl group or a C₁₋₄-acyl group, particularly acetyl group. The molecules in which R² represents an acyl group, particularly an acetyl group, can be easily prepared from the corresponding tocopherol of formula (I) by esterification; conversely, the phenolic compound can be obtained from the corresponding ester by ester hydrolysis.

It is mostly preferred that R² is H.

As 3,4-dehydrotocopherol of formula (II) is present in tocopherol, the 3,4-dehydrotocopherol of formula (II) will also be protected by the same protecting group as the corresponding tocopherol when the tocopherol of formula (I) is converted to protected tocopherol of formula (I) by the reaction with the protecting agent.

The tocopherol or protected tocopherol of formula (I), containing as an impurity the 3,4-dehydrotocopherol or protected 3,4-dehydrotocopherol of formula (II), respectively, is contacted with an acid activated bleaching earth.

### Acid activated bleaching earth

The term "bleaching earth" (German: "Bleicherde") is known to the person skilled in the art to be a collective name for a group of aluminium and/or magnesium silicates comprising water (Römpp, Lexikon der Chemie, Bleicherde, 10. Auflage, Stuttgart, 1996, page 469). Particularly suitable bleaching earths are attapulgite, bentonite and montmorillonite.

"Acid activated bleaching earths" are bleaching earths which are treated by strong mineral acids. This acid treatment leads to ion exchange in the bleaching earth. Due to this ion-exchange, i.e. acid activation, the properties of the bleaching earths are strongly modified.

The acid activated bleaching earth is preferably an acid activated bentonite. Particularly suitable are acid activated bleaching earths which are commercialized under the trademark TONSIL® by Süd-Chemie.

In a further embodiment the acid activated bleaching earths are those commercialized as activated clays F-20 and F-20X by BASF.

The acid activated bleaching earth has preferably a specific surface area (BET) between 150 and 400 m²/g, preferably between 250 and 375 m²/g, more preferably between 300 and 350 m²/g. The specific surface area is measured according to the method BET (Brunauer, Emmett und Teller) which is a commonly known method for determination of surface areas of solid particles by physical adsorption of gas, particularly as described in ISO 9277.

Particularly a 10 % by weight suspension in water has a pH of between 1.5 and 5, preferably between 1.5 and 4, measured at 25°C and after filtration.

The acidity of the acid activated bleaching earth, measured as mg KOH/g, is preferably in the range of 0.1 to 22, more preferably in the range of 0.2 to 21, even more preferably in the range of 0.3 to 20.

Furthermore, it is preferred that the acid activated bleaching earth has after drying at 110°C during 2 hours a residual amount of CaO of less than 1.5 % by weight, particularly between 0.001 and 0.8 % by weight.

Particularly suitable are the products of the trademark TONSIL® SUPREME such as TONSIL® SUPREME 110 FF, TONSIL® SUPREME 112 FF, TONSIL® SUPREME 114 FF, TONSIL® SUPREME 115 FF and TONSIL® SUPREME 118 FF and TONSIL® Optimum 210 FF allowing a fast separation, particularly a fast filtration. Furthermore suitable are the products sold as activated clays F-20 and F-20X by BASF. It has been observed that TONSIL® SUPREME 115 FF, activated clay F-20, and F-20X are particularly suitable for the purpose of the present invention.

Acid activated bleaching earths are very interesting in view of toxicological aspects. Acid activated bleaching earths are particularly suitable for the treatment of food and feed ingredients, since for a long time already, acid activated bleaching earths are used on an industrial scale for the treatment of vegetable oils and fats. The acid activated bleaching earths can also be easily removed completely from the tocopherols.

Tocopherol of formula (I) is a known product as such and is part of vitamin E. It can be produced in different ways as disclosed in Ullmann's Encyclopedia of Industrial Chemistry, Release 2010, 7th Edition, "Vitamins", pages 43 - 47 or in WO 2005/054223 A2.

The preferred route of synthesis is the condensation of the corresponding methyl-, dimethyl- or trimethylhydoquinone of formula (III) with phytol or isophytol.

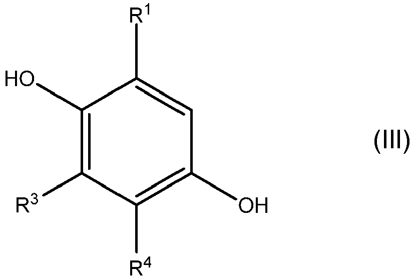

For this condensation a series of catalysts may be used such as ZnCl₂/mineral acid, BF₃/AlCl₃, Fe/HCl, trifluoroacetic acid or boric acid/carboxylic acid as well as indium (III) or scandium (III) salts as disclosed by WO 2005/121115 A1. Furthermore suitable catalysts are heteropoly acids, particularly 12-tungstophosphoric or 12-tungstosilicic acid.

Said reactions are not stereospecific and, hence, a mixture of isomers of tocopherol of formula (I) is obtained. This is reflected by the use of the * in the formulae of this document. Due to the fact that there are 3 chiral centres in compounds of formula (I) there exist 8 individual isomers. Such a product being a mixture of R- and S- configuration at each chiral centre (2, 4' and 8') indicated by * in this document is also called (*all*-*rac*)-tocopherol or (*all*-*rac*)-tocopheryl acetate (in case the phenol protection group is an acetyl group).

In view of industrial interest and volume of production it is preferred that tocopherol of formula (I) is (*all*-*rac*)-tocopherol, particularly (*all*-*rac*)--α-tocopherol and that the protected tocopherol of formula (I) is *(all-rac)-*tocopheryl acetate, particularly (*all*-*rac*)-α-tocopheryl acetate.

Since the different isomers have different bioactivity it is in the interest to synthesize specifically a desired isomer having high bioactivity. (2R, 4'R, 8'R)-tocopherol isomers have shown to exhibit a particularly high bioactivity. There exist such stereoselective syntheses which yield to either specific isomers or mixtures with less isomers only. For example, natural phytol can be used for the above synthesis. Natural phytol consists only of the R,R-isomer and, hence, is isomerically pure. Therefore, the above reaction leads to a mixture of (2R, 4'R, 8'R)-tocopherol and (2S, 4'R, 8'R)-tocopherol, also known as 2-*ambo*-tocopherol. However, as natural phytol, or isophytol, is commercially available only in rather small amounts and is rather expensive, the potential of using natural phytol, or isophytol, for industrial scale synthesis of tocopherols is rather limited.

Is has been found, however, that by using specific chiral iridium complexes, asymmetrical hydrogenation of alkenes can be achieved. The chiral iridium complexes disclosed in WO 2006/066863 A1 as well as in the pending application EP11169784.3 are particularly suited for this purpose. By this use of asymmetrical hydrogenation 2*-ambo-*tocopherol can be obtained, the isomers of which can be separated much more easily, more efficiently and particularly in a much more cost effective way than isomeric mixtures obtained by traditional processes.

Therefore, it is preferred in another embodiment that the tocopherol of formula (I) is a mixture of (2R, 4'R, 8'R)-tocopherol and (2S, 4'R, 8'R)-tocopherol or that the protected tocopherol of formula (I) is a mixture of (2R, 4'R, 8'R)-tocopheryl acetate and (2S, 4'R, 8'R)-tocopheryl acetate.

More preferred, the tocopherol or protected tocopherol of formula (I) is (2R, 4'R, 8'R)-tocopherol or (2R, 4'R, 8'R)-tocopheryl acetate.

Most preferred, the tocopherol or protected tocopherol of formula (I) is (2R, 4'R, 8'R)-α-tocopherol or (2R, 4'R, 8'R)-α-tocopheryl acetate.

Preferred are the following combinations of R¹, R³ and R⁴:

R¹ = R³ = R⁴ = CH₃

or

R¹ = R⁴ = CH₃, R³ = H

or

R¹ = H, R³ = R⁴ = CH₃

or

R¹ = R³ = H, R⁴ = CH₃

Most preferred is the combination R¹ = R³ = R⁴ = CH₃.

Therefore, preferably the tocopherol or protected tocopherol of formula (I) is selected from the group consisting of α-tocopherol, α-tocopheryl acetate, β-tocopherol, β-tocopheryl acetate, γ-tocopherol, γ-tocopheryl acetate, δ-tocopherol and δ-tocopheryl acetate, particularly α-tocopherol or α-tocopheryl acetate.

It is preferred that only one type of tocopherol or protected tocopherol of formula (I), is present when the contacting with the acid activated bleaching earth is performed. In other words it is preferred that no mixtures of different tocopherols or protected tocopherols with different groups R¹, R³ and R⁴, particularly no mixtures comprising α-tocopherol and γ-tocopherol or no mixtures comprising α-tocopheryl acetate and γ-tocopheryl acetate, are contacted with acid activated bleaching earth.

A key element of the present invention is the interaction of acid activated bleaching earth with the tocopherol or protected tocopherol of formula (I) in the form of neat or in form of highly concentrated solution tocopherol or protected tocopherol. This interaction requires a physical contact of the tocopherol or the protected tocopherol with the acid activated bleaching earth. It is desired that the contact is thorough. Hence, any possibility allowing a thorough contact between the surface of the acid activated bleaching earth with the tocopherol or protected tocopherol of formula (I) can be principally used.

A preferred possibility for such a thorough contact, which is particularly suitable for the purpose of the invention, is when the contacting is realized by adding the acid activated bleaching earth to the tocopherol or protected tocopherol of formula (I) and stirring. This leads to a suspension of acid activated bleaching earth in a liquid phase comprising the tocopherol or protected tocopherol of formula (I). This can be realized in a continuous or in a batchwise manner in a stirred tank or a cascade of this reactor types.

There exist also other possibilities of contacting. The contact with the acid activated bleaching earth can be realized for example by a flow of the tocopherol or the protected tocopherol over the acid activated bleaching earth being immobilized on a carrier such as a plate or a wall or by a flow through a column filled with acid activated bleaching earth. In order to increase the time of contact between activated bleaching earth and the tocopherol or the protected tocopherol, it is advisable to use a flow in a continuous loop. However, it has been observed that particularly the use of a fixed bed reactor is disadvantageous as the contact is not sufficient enough to allow an industrially effective process at an acceptable pressure drop.

It is preferred that the contacting is performed under inert conditions, particularly under nitrogen.

Furthermore, it has been shown that the contact is particularly efficient when the contacting is done at elevated temperature, particularly at a temperature of between 80 and 160 °C, preferably between 100 and 150°C.

It is further preferred that the time of contact is between 30 and 300 minutes, preferably between 45 and 180 minutes, more preferably between 60 and 120 minutes.

Particularly preferred is when the contact is realized at an absolute pressure of 0.01 - 1013 mbar, preferably 10 - 500 mbar, more preferably 20 - 200 mbar.

The step of contacting can be realized in a continuous or in a batch process

It has been observed that it is preferred that the weight ratio of acid activated bleaching earth to tocopherol or protected tocopherol of formula (I) is between 0.5 / 100 and 5 / 100, particularly is between 1 / 100 and 3 / 100, preferably between 1 / 100 and 2 / 100. For example, using 5 g acid activated bleaching earth per 100 g tocopherol or protected tocopherol of formula (I) leads to a weight ratio of acid activated bleaching earth to tocopherol or protected tocopherol of formula (I) of 5 / 100.

The tocopherol or protected tocopherol of formula (I) is contacted as such, i.e. in the form of neat tocopherol or protected tocopherol of formula (I), or in the form of a solution comprising more than 60 %, preferably more than 90 %, particularly more than 95 %, by weight of tocopherol or protected tocopherol of formula (I), with the acid activated bleaching earth. For the solution a non-polar solvent, particularly selected from the group consisting of hexane, heptane, xylene and toluene is preferred.

The use of solvents to yield a solution is advantageous in view of lowering the viscosity of the tocopherol or the protected tocopherol of formula (I). A lower viscosity enables a better contact, particularly at lower temperatures and an easier and faster separation from the acid activated bleaching earth.

As the method of invention focuses particularly to tocopherols and protected tocopherols originating from chemical synthetic processes, any solution should also not comprise any triglycerides or any fatty acids, particularly as they occur in vegetable oils.

In the case where the method relates to the protected tocopherol of formula (I) there exist two major possibilities in the sequences of steps.

In one embodiment of the invention the tocopherol of formula (I) is first reacted with the protecting agent to yield the protected tocopherol of formula (I) and then contacted with the acid activated bleaching earth.

In another embodiment of the invention the tocopherol of formula (I) is first contacted with the acid activated bleaching earth and then reacted with the protecting agent to yield the protected tocopherol of formula (I).

The latter of these two embodiments just mentioned is more preferred as it leads to a lower amount of free tocopherol in the final product (the protected tocopherol of formula (I)) due to ester cleavage.

After contacting of the tocopherol or protected tocopherol of formula (I) with an acid activated bleaching earth the acid activated bleaching earth is separated from the tocopherol or protected tocopherol of formula (I) in a further step. The separation can be achieved for example by filtering off the acid activated bleaching earth after the contacting or by centrifugation.

### Reduction of amount of dehydrotocopherol or protected dehydrotocopherol of formula (II)

The present invention leads to tocopherol or protected tocopherol of formula (I) with a reduced content of dehydrotocopherol or protected dehydrotocopherol of formula (II).

The amount of dehydrotocopherol or protected dehydrotocopherol of formula (II) is reduced at least by 20 %, particularly at least 28 %, preferably at least 40 %, more preferably at least 50 %, by weight compared to the amount of the corresponding dehydrotocopherol or protected dehydrotocopherol of formula (II) measured in tocopherol or protected tocopherol of formula (I) which has not been contacted with acid activated bleaching earth *("comparative tocopherol or comparative protected tocopherol"*)*.*

It is important to stress that this definition relies on the comparison of two tocopherols or protected tocopherols of formula (I) being synthesized and treated in a completely identical manner except that the step of contacting tocopherol or protected tocopherol of formula (I) with an acid activated bleaching earth has not been performed for the *comparative tocopherol or comparative protected tocopherol.*

It has been observed that a variety of parameters have a more or less pronounced effect on the degree of reduction of the amount of dehydrotocopherol or protected dehydrotocopherol of formula (II), such as temperature, pressure, time of contacting or origin of starting materials.

The amount of dehydrotocopherol or protected dehydrotocopherol of formula (II) can be determined by gas chromatography.

Particularly the amount of dehydrotocopherol of formula (I) is measured by means of a gas chromatograph (Agilent, model 6850) using an autosampler, a FID detector and a column HP Ultra 2 (Crosslinked 5 % phenylsilanol, 95 % methylsilanol; length 25 m, 0.32 mm internal diameter, 0.52 µm film) and the following parameters: carrier gas: H₂, constant flow: 2.5ml/min, injector temperature: 280°C, injection volume: 1.0 µl, temperature program: 180°C to 280°C rate 20.0 °C/min, detector temperature: 300°C. The sample was injected as a solution of 4 mg/ml in heptane.

In case of 3,4-dehydro-α-tocopherol, the peak at a retention time of 11.5 minutes was identified to be 3,4-dehydro-α-tocopherol.

The amount was determined by calibration with an authentic reference substance to be detected, such as 3,4-dehydro-α-tocopherol.

Particularly the amount of protected dehydrotocopherol of formula (II) is measured by means of a gas chromatograph (HP 6890) using an autosampler, a FID detector and a fused silica column (Rtx-5SilMS; length 30 m, 0.28 mm internal diameter, 0.5 µm film) and the following parameters: carrier gas: helium, constant flow: 1.5 ml/min, injector temperature: 300°C, injection volume: 1.0 µl, temperature program: 150°C to 350°C rate 5 °C/min, detector temperature: 330°C. The sample was injected as a solution of 20 mg/ml in pyridine/ N,O-Bis(trimethylsilyl)trifluoroacetamide 1:1.

In case of 3,4-dehydro-α-tocopheryl acetate, the peak at a retention time of 33.7 minutes was identified to be 3,4-dehydro-α-tocopheryl acetate.

The amount was determined by calibration with an authentic reference substance to be detected, such as 3,4-dehydro-α-tocopheryl acetate.

With the method of invention, a residual amount of dehydrotocopherol or protected dehydrotocopherol of formula (II) of < 0.21 % by weight, particularly < 0.18 % by weight, based on the weight of tocopherol or protected tocopherol of formula (I) can be achieved. Within the range indicated above, by using a higher weight ratio of acid activated bleaching earth to tocopherol or protected tocopherol of formula (I) it has been found that the residual amount of dehydrotocopherol or protected dehydrotocopherol of formula (II) is even below 0.12 %. By further optimizing the present method, it is possible to reduce the amount of this impurity even further, so that the residual amount of dehydrotocopherol or protected dehydrotocopherol of formula (II) is even below 0.05 %.

In a preferred embodiment, a residual amount of 3,4-dehydro-α-tocopherol or 3,4-dehydro-α-tocopheryl acetate of < 0.21 % by weight, particularly < 0.18 % by weight, preferred < 0.12 % by weight, particularly < 0.05 % by weight, based on the weight of (*all*-*rac*)-α-tocopherol, respectively of (*all*-*rac*)-α-tocopheryl acetate is achieved.

Furthermore, it has been found that also after long-term-storage of several months (up to 6 months, resp. 9 months) at a temperature of 25°C or 40°C the amount of dehydrotocopherol or protected dehydrotocopherol of formula (II) remains at this low level and particularly does not increase.

Finally, it is also important to stress that this method does not need any additive which needs to remain continuously in the tocopherol or protected tocopherol of formula (I) which eventually would restrict the possible fields of applications of the tocopherols or protected tocopherols because of toxicological doubts or limitations of such an additive. The current method, however, has no such limitations and is therefore optimal for using tocopherols or protected tocopherols in pharma, food or feed applications.

In a further aspect the invention relates to the use of an acid activated bleaching earth for the purification of tocopherol or protected tocopherol of formula (I) in the form of neat tocopherol or protected tocopherol of formula (I), or in the form of a solution comprising more than 60 % by weight of tocopherol or protected tocopherol of formula (I). The tocopherol or protected tocopherol of formula (I) has been already described above in detail.

Accordingly, also the tocopherol or protected tocopherol of formula (I) with a reduced content of dehydrotocopherol or protected dehydrotocopherol of formula (II), respectively, prepared by the method as described above, represents a further aspect of the current invention.

The tocopherol or protected tocopherol of formula (I) can be used in different fields. Preferably it can be used in the field of pharma or food or feed.

Therefore, particularly the process of preparing a food or feed composition with a reduced content of dehydrotocopherol or protected dehydrotocopherol of formula (II) comprising the steps
a) preparing a tocopherol or protected tocopherol of formula (I) with a reduced content of dehydrotocopherol or protected dehydrotocopherol of formula (II) according to the method as described above;
   and
b) adding tocopherol or protected tocopherol of formula (I) with a reduced content of dehydrotocopherol or protected dehydrotocopherol of formula (II) yielding from step a) to at least one dietary supplement or food or feed ingredient;
represents a further aspect of the present invention.

### Examples

### Comparative example Ref. 1:

100 kg (*all*-*rac*)-α-tocopherol was added to the reactor. In this reactor the sample was stirred at an absolute pressure of 50-60 mbar under nitrogen during 90 minutes at 130 °C. Afterwards the sample was rectified under vacuum by a rectification column.

### Example 1

As in comparative example *Ref. 1,* 100 kg (*all*-*rac*)-α-tocopherol was added to the reactor. In this reactor 2 kg of TONSIL® SUPREME 115 FF has been added and stirred at an absolute pressure of 50-60 mbar under nitrogen during 90 minutes at 130 °C. Afterwards the acid activated bleaching earth was filtered off. Then the sample was rectified under identical conditions as used for comparative example *Ref. 1.*

### Quantification of 3,4-dehydro-α-tocopherol

The amount of 3,4-dehydro-α-tocopherol is measured by means of a gas chromatograph (Agilent, model 6850) using an autosampler, a FID detector and a column HP Ultra 2 (Crosslinked 5 % phenylsilanol, 95 % methylsilanol; length 25 m, 0.32 mm internal diameter, 0.52 µm film) and the following parameters: carrier gas: H₂, constant flow: 2.5 ml/min, injector temperature: 280°C, injection volume: 1.0 µl, temperature program: 180°C to 280°C rate 20.0 °C/min, detector temperature: 300°C. The sample was injected as a solution of 4 mg/ml in heptane. The peak maximum of 3,4-dehydro-α-tocopherol was detected at a retention time of 11.5 minutes. The amount was determined by calibration with an authentic reference substance of 3,4-dehydro-α-tocopherol.

### Storage of samples Ref. 1 and 1 & Results:

A sample of 2 liters each of ***Ref.1*** and 1 has been taken and filled under argon into separate flasks and sealed under argon. An analytical sample has been taken from the 2 liter samples before filling and the amount of 3,4-dehydro-α-tocopherol therein has been determined (storage time: 0 month).

Half of the flasks containing ***Ref.1*** or 1 have been stored at 25°C and 65 % relative humidity, and the other half at 40°C and 75 % relative humidity. After 1 month, 3 months, 6 months, 9 months and 12 months of storage under these conditions, one flask each was taken out of the storage and an analytical sample has been taken and the amount of 3,4-dehydro-α-tocopherol therein has been determined accordingly.

The values obtained are given in tables 1 and 2.

**Table 1. Amounts of 3,4-dehydro-α-tocopherol during long term storage at 25°C.**

| | Content of 3,4-dehydro-α-tocopherol [% by weight] in the sample after storage at 25 °C / 65 % relative humidity after | | | | |
|---|---|---|---|---|---|
| | 0 month | 1 month | 3 months | 6 months | 12 months |
| ***Ref.1*** | 0.25 | n.d.¹ | 0.25 | 0.26 | 0.27 |
| ***1*** | 0.18 | n.d.¹ | 0.19 | 0.18 | 0.19 |
| Reduction by | 28% | n.d.¹ | 24% | 31% | 30% |

| | | | | | |
|---|---|---|---|---|---|
| ¹ n.d.= not determined | | | | | |

**Table 2. Amounts of 3,4-dehydro-α-tocopherol during long term storage at 40°C.**

| | Content of 3,4-dehydro-α-tocopherol [% by weight] in the sample after storage at 40°C / 75 % relative humidity after | | | | |
|---|---|---|---|---|---|
| | 0 month | 1 month | 3 months | 6 months | 9 months |
| ***Ref.1*** | 0.25 | 0.27 | 0.27 | 0.26 | 0.27 |
| ***1*** | 0.18 | 0.19 | 0.18 | 0.19 | 0.15 |
| Reduction by | 28% | 30% | 33% | 27% | 44% |

### Comparative example Ref. 2:

A sample of 400 g (*all*-*rac*)-α-tocopherol, different from the one used for example ***1*,** has been put in a lab reactor and stirred under nitrogen at an absolute pressure of 50 mbar during 90 minutes at 110 °C.

### Example 2

A sample of 400 g (*all*-*rac*)-α-tocopherol, as used for comparative example *Ref. 2,* has been put in a lab reactor. In this reactor 8 g of TONSIL® SUPREME 115 FF have been added and stirred under nitrogen at an absolute pressure of 50 mbar during 90 minutes at 110 °C. Afterwards the acid activated bleaching earth was filtered off.

### Storage samples Ref. 2 and 2 & Results:

An analytical sample of *Ref.2* and ***2*** has been taken and the amount of 3,4-dehydro-α-tocopherol therein has been determined (storage time: 0 month).

*Ref.2* and ***2*** then have been filled under argon into separate flasks and sealed under argon. These flasks have been stored at 40°C and 75 % relative humidity. After 1 month, 3 months, 6 months and 9 months of storage under these conditions, one flask was taken out of the storage and an analytical sample has been taken and the amount of 3,4-dehydro-α-tocopherol has been determined accordingly. The method of determining the amount of 3,4-dehydro-α-tocopherol therein is as mentioned in example 1.

The values are given in table 3.

**Table 3. Amounts of 3,4-dehydro-α-tocopherol during long term storage at 40°C.**

| | Content of 3,4-dehydro-α-tocopherol [% by weight] in the sample after storage at 40°C / 75 % relative humidity after | | | | |
|---|---|---|---|---|---|
| | 0 month | 1 month | 3 months | 6 months | 9 months |
| ***Ref.2*** | 0.23 | 0.21 | 0.22 | 0.24 | 0.26 |
| ***2*** | 0.11 | 0.10 | 0.10 | 0.13 | 0.14 |
| Reduction | 52% | 52% | 55% | 46% | 46% |

One can clearly derive from the data in tables 1-3 the efficiency of removal of 3,4-dehydro-α-tocopherol by treatment with acid activated bleaching earth.

### Comparative example Ref. 3:

In a cascade of 3 stirred vessels, a stream of 100 kg/h of (*all*-*rac*)-α-tocopherol was fed and stirred at 140 °C and at an absolute pressure of 50 mbar. The mean residence time in the 3-reactor cascade was 90 minutes.

100 kg of the so treated (*all*-*rac*)-α-tocopherol was rectified over a distillation column and subsequently esterified by adding 72.3 kg acetic anhydride and 0.65 kg pyridine at total reflux (atmospheric pressure) to form (*all*-*rac*)-α-tocopherol acetate. After distilling off the excess of acetic anhydride, pyridine, and the acetic acid the (*all*-*rac*)-α-tocopheryl acetate was further rectified over a rectification column (sump temperature = 275°C). The content of 3,4-dehydro-α-tocopheryl acetate in the *comparative* (*all*-*rac*)-*α*-*tocopheryl acetate **Ref. 3,*** obtained by this manner, was measured using the method indicated below.

### Example 3

In the same cascade of 3 stirred vessels, as used for the comparative example ***Ref.3,*** to a stream of 100 kg/h of (*all*-*rac*)-α-tocopherol, as used for comparative example ***Ref.3,*** continuously 1.6 kg/h of TONSIL® SUPREME 115 FF was added and stirred at 140 °C and at an absolute pressure of 50 mbar. At the exit the acid activated bleaching earth was continuously filtered. The mean residence time for the contacting in the same cascade of 3 vessels was 90 minutes.

100 kg of the so purified (*all*-*rac*)-α-tocopherol was rectified in a column, esterified and rectified identically to the comparative example ***Ref.3*** to yield (*all*-*rac*)-α-tocopheryl acetate. Except the contacting with the acid activated bleaching earth, the example ***3*,** hence, has been prepared identically as the comparative example ***Ref. 3.***

The content of 3,4-dehydro-α-tocopheryl acetate in the *(all-rac)-*α-tocopheryl acetate ***3*,** obtained by this manner, was measured using the method indicated below.

### Quantification of 3,4-dehvdro-α-tocopheryl acetate

The amount of 3,4-dehydro-α-tocopheryl acetate was measured by means of a gas chromatograph (HP 6890) using an autosampler and a FID detector and a fused silica column (Rtx-5SilMS; length 30 m, 0.28 mm internal diameter, 0.5 µm film) and the following parameters: carrier gas: helium, constant flow: 1.5ml/min, injector temperature: 300°C, injection volume: 1.0 µl, temperature program: 150°C to 350°C rate 5 °C/min), detector temperature: 330°C). The sample was injected as a solution of 20 mg/ml in pyridine/N,O-Bis(trimethylsilyl)trifluoroacetamide 1:1. The peak maximum of 3,4-dehydro-α-tocopheryl acetate was detected at a retention time of 33.7 minutes. The amount was determined by calibration with an authentic reference substance of 3,4-dehydro-α-tocopheryl acetate.

The average of the three measurements as well as the standard deviation of the corresponding samples is given in table 4.

**Table 4. Amounts of 3,4-dehydro-α-tocopheryl acetate.**

| Sample | |
|---|---|
| ***Ref. 3*** [% by weight] | 0.11 ± 0.01 |
| ***3*** [% by weight] | 0.057 ± 0.006 |
| Reduction by | 48% |

## Claims

1. Method of preparing a tocopherol or protected tocopherol of formula (I) with a reduced content of dehydrotocopherol or protected dehydrotocopherol of formula (II) comprising the step of contacting tocopherol or protected tocopherol of formula (I), in the form of neat tocopherol or protected tocopherol of formula (I) or in the form of a solution comprising more than 60 % by weight of tocopherol or protected tocopherol of formula (I), with an acid activated bleaching earth 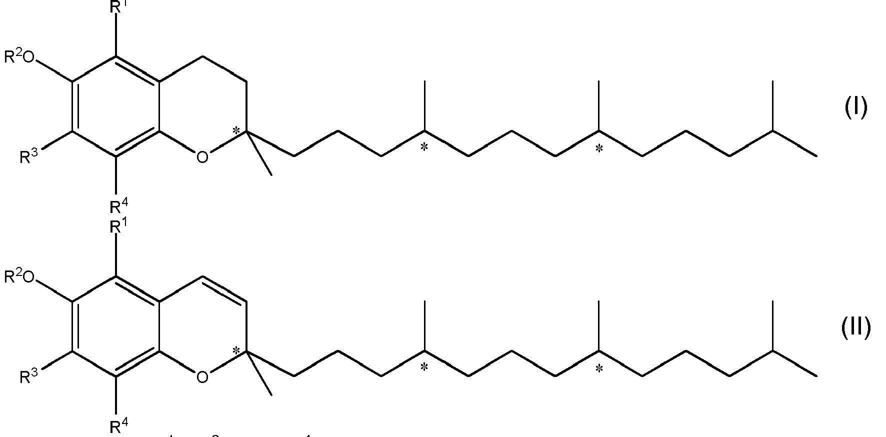
wherein R¹, R³ and R⁴ are independently from each other hydrogen or methyl groups;
R² represents hydrogen or a phenol protection group;
and wherein each * represents an individual chiral centre;
**characterized in that**
the reduction of dehydrotocopherol or protected dehydrotocopherol of formula (II) is at least 20 % by weight compared to the amount of the corresponding dehydrotocopherol or protected dehydrotocopherol of formula (II) measured in tocopherol or protected tocopherol of formula (I) which has not been contacted with acid activated bleaching earth;
and **in that** the phenol protection group R² forms with the rest of the molecule a chemical functionality which is selected from the group consisting of ester, ether or acetal.

2. Method according to claim 1 **characterized in that** the acid activated bleaching earth is an acid activated bentonite.

3. Method according to anyone of the preceding claims **characterized in that** the acid activated bleaching earth has a specific surface area (BET) between 150 and 400 m²/g, preferably between 250 and 375 m²/g, more preferably between 300 and 350 m²/g.

4. Method according to anyone of the preceding claims **characterized in that** R² is H.

5. Method according to anyone of the preceding claims **characterized in that** tocopherol or protected tocopherol of formula (I) is selected from the group consisting of α-tocopherol, α-tocopheryl acetate, β-tocopherol, β-tocopheryl acetate, γ-tocopherol, γ -tocopheryl acetate, δ-tocopherol and δ-tocopheryl acetate, particularly α-tocopherol or α-tocopheryl acetate.

6. Method according to anyone of the preceding claims **characterized in that** the contacting is done at a temperature of between 80 and 160 °C, preferably between 100 and 150°C.

7. Method according to anyone of the preceding claims **characterized in that** the contacting is realized **in that** the acid activated bleaching earth is added to the tocopherol or protected tocopherol of formula (I) and stirred.

8. Method according to anyone of the preceding claims **characterized in that** weight ratio of acid activated bleaching earth to tocopherol or protected tocopherol of formula (I) is between 0.5 / 100 and 5 / 100, particularly is between 1 / 100 and 3 / 100, preferably between 1 / 100 and 2 / 100.

9. Method according to anyone of the preceding claims **characterized in that** the tocopherol or protected tocopherol of formula (I) in the form of neat tocopherol or protected tocopherol of formula (I) does not comprise any triglycerides or any fatty acids.

10. Process of preparing a food or feed composition with a reduced content of dehydrotocopherol or protected dehydrotocopherol of formula (II) comprising the steps of
a) preparing a tocopherol or protected tocopherol of formula (I) with a reduced content of dehydrotocopherol or protected dehydrotocopherol of formula (II) according to the method of claims 1 - 9;
and
b) adding tocopherol or protected tocopherol of formula (I) with a reduced content of dehydrotocopherol or protected dehydrotocopherol of formula (II) yielding from step a) to at least one dietary supplement or food or feed ingredient.

## Patentansprüche

1. Verfahren zur Herstellung eines Tocopherols oder geschützten Tocopherols der Formel (I) mit einem verminderten Gehalt an Dehydrotocopherol oder geschütztem Dehydrotocopherol der Formel (II), umfassend den Schritt des Inkontaktbringens von Tocopherol oder geschütztem Tocopherol der Formel (I), in Form von Tocopherol oder geschütztem Tocopherol der Formel (I) als Substanz oder in Form einer Lösung, die mehr als 60 Gew.-% Tocopherol oder geschütztes Tocopherol der Formel (I) umfasst, mit einer säureaktivierten Bleicherde, 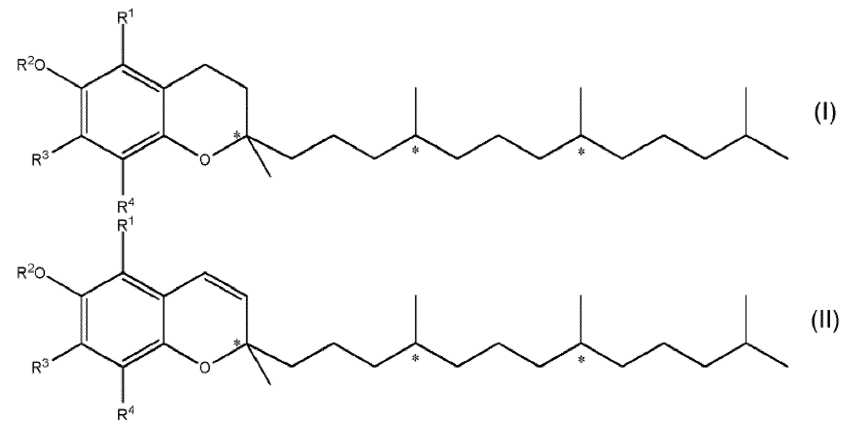
wobei R¹, R³ und R⁴ unabhängig voneinander Wasserstoff oder Methylgruppen bedeuten;
R² Wasserstoff oder eine Phenolschutzgruppe bedeutet;
und wobei jedes * ein einzelnes Chiralitätszentrum bedeutet;
**dadurch gekennzeichnet, dass**
die Verminderung an Dehydrotocopherol oder geschütztem Dehydrotocopherol der Formel (II) mindestens 20 Gew.-% im Vergleich zu der Menge an entsprechendem Dehydrotocopherol oder geschütztem Dehydrotocopherol der Formel (II), das in Tocopherol oder geschütztem Tocopherol der Formel (I), das nicht mit säureaktivierter Bleicherde in Kontakt gebracht worden ist, gemessen wurde, beträgt;
und dass die Phenolschutzgruppe R² mit dem Rest des Moleküls eine chemische Funktionalität bildet, die aus der Gruppe bestehend aus Ester, Ether oder Acetal ausgewählt ist.

2. Verfahren nach Anspruch 1, **dadurch gekennzeichnet, dass** es sich bei der säureaktivierten Bleicherde um einen säureaktivierten Bentonit handelt.

3. Verfahren nach einem der vorhergehenden Ansprüche, **dadurch gekennzeichnet, dass** die säureaktivierte Bleicherde eine spezifische Oberfläche (BET) zwischen 150 und 400 m²/g, vorzugsweise zwischen 250 und 375 mg²/g, stärker bevorzugt zwischen 300 und 350 m²/g, aufweist.

4. Verfahren nach einem der vorhergehenden Ansprüche, **dadurch gekennzeichnet, dass** R² H bedeutet.

5. Verfahren nach einem der vorhergehenden Ansprüche, **dadurch gekennzeichnet, dass** Tocopherol oder geschütztes Tocopherol der Formel (I) aus der Gruppe bestehend aus α-Tocopherol, α-Tocopherylacetat, β-Tocopherol, β-Tocopherylacetat, γ-Tocopherol, γ-Tocopherylacetat, δ-Tocopherol und δ-Tocopherylacetat, insbesondere α-Tocopherol oder α-Tocopherylacetat, ausgewählt ist.

6. Verfahren nach einem der vorhergehenden Ansprüche, **dadurch gekennzeichnet, dass** das Inkontaktbringen bei einer Temperatur zwischen 80 und 160°C, vorzugsweise zwischen 100 und 150°C, erfolgt.

7. Verfahren nach einem der vorhergehenden Ansprüche, **dadurch gekennzeichnet, dass** das Inkontaktbringen durch Zusetzen der säureaktivierten Bleicherde zu dem Tocopherol oder geschütztem Tocopherol der Formel (I) und Rühren erfolgt.

8. Verfahren nach einem der vorhergehenden Ansprüche, **dadurch gekennzeichnet, dass** das Gewichtsverhältnis von säureaktivierter Bleicherde zu Tocopherol oder geschütztem Tocopherol der Formel (I) zwischen 0,5/100 und 5/100, insbesondere zwischen 1/100 und 3/100, vorzugsweise zwischen 1/100 und 2/100, beträgt.

9. Verfahren nach einem der vorhergehenden Ansprüche, **dadurch gekennzeichnet, dass** das Tocopherol oder geschützte Tocopherol der Formel (I) in Form von Tocopherol oder geschütztem Tocopherol der Formel (I) in Substanz keine Triglyceride oder Fettsäuren umfasst.

10. Verfahren zur Herstellung einer Nahrungsmittel- oder Futterzusammensetzung mit einem verminderten Gehalt an Dehydrotocopherol oder geschütztem Dehydrotocopherol der Formel (II), umfassend die folgenden Schritte:
a) Herstellen eines Tocopherols oder geschützten Tocopherols der Formel (I) mit einem verminderten Gehalt an Dehydrotocopherol oder geschütztem Dehydrotocopherol der Formel (II) nach dem Verfahren der Ansprüche 1 - 9; und
b) Zusetzen von aus Schritt a) erhaltenem Tocopherol oder geschütztem Tocopherol der Formel (I) mit einem verminderten Gehalt an Dehydrotocopherol oder geschütztem Dehydrotocopherol der Formel (II) zu mindestens einem Nahrungsmittelzusatzstoff oder Nahrungsmittel- oder Futterbestandteil.

## Revendications

1. Procédé de préparation d'un tocophérol ou tocophérol protégé de formule (I) avec une teneur réduite en déhydrotocophérol ou déhydrotocophérol protégé de formule (II) comprenant l'étape consistant à mettre en contact le tocophérol ou tocophérol protégé de formule (I), sous la forme de tocophérol ou tocophérol protégé de formule (I) pur ou sous la forme d'une solution comprenant plus de 60 % en poids de tocophérol ou tocophérol protégé de formule (I), avec une terre décolorante activée par acide 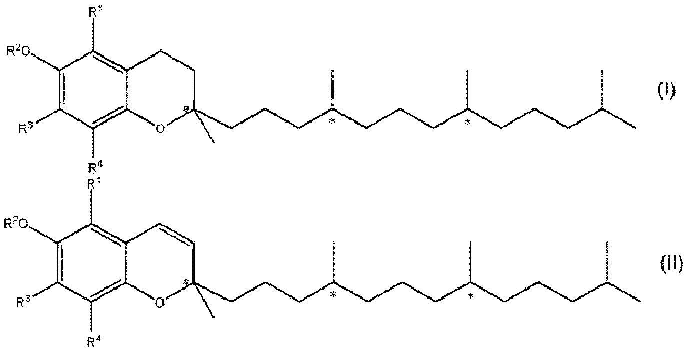
dans lesquels R¹, R³ et R⁴ représentent indépendamment les uns des autres un hydrogène ou un groupe méthyle ; R² représente un hydrogène ou un groupe de protection du phénol ;
et dans lesquels chaque * représente un centre chiral individuel ;
**caractérisé en ce que**
la réduction du déhydrotocophérol ou déhydrotocophérol protégé de formule (II) est d'au moins 20 % en poids par rapport à la quantité du déhydrotocophérol ou déhydrotocophérol protégé de formule (II) correspondant mesurée dans le tocophérol ou tocophérol protégé de formule (I) qui n'a pas été mis en contact avec une terre décolorante activée par acide ;
et **en ce que** le groupe de protection du phénol R² forme avec le reste de la molécule une fonctionnalité chimique qui est choisie dans le groupe constitué par un ester, un éther et un acétal.

2. Procédé selon la revendication 1 **caractérisé en ce que** la terre décolorante activée par acide est une bentonite activée par acide.

3. Procédé selon l'une quelconque des revendications précédentes **caractérisé en ce que** la terre décolorante activée par acide a une surface spécifique (BET) comprise entre 150 et 400 m²/g, de préférence entre 250 et 375 m²/g, mieux encore entre 300 et 350 m²/g.

4. Procédé selon l'une quelconque des revendications précédentes **caractérisé en ce que** R² représente H.

5. Procédé selon l'une quelconque des revendications précédentes **caractérisé en ce que** le tocophérol ou tocophérol protégé de formule (I) est choisi dans le groupe constitué par l'α-tocophérol, l'acétate d'α-tocophéryle, le β-tocophérol, l'acétate de β-tocophéryle, le γ-tocophérol, l'acétate de γ-tocophéryle, le δ-tocophérol et l'acétate de δ-tocophéryle, en particulier l'α-tocophérol ou l'acétate d'α-tocophéryle.

6. Procédé selon l'une quelconque des revendications précédentes **caractérisé en ce que** la mise en contact est effectuée à une température comprise entre 80 et 160 °C, de préférence entre 100 et 150 °C.

7. Procédé selon l'une quelconque des revendications précédentes **caractérisé en ce que** la mise en contact est réalisée de telle sorte que la terre décolorante activée par acide est ajoutée au tocophérol ou tocophérol protégé de formule (I) et agitée.

8. Procédé selon l'une quelconque des revendications précédentes **caractérisé en ce que** le rapport pondéral entre la terre décolorante activée par acide et le tocophérol ou tocophérol protégé de formule (I) se situe entre 0,5/100 et 5/100, en particulier se situe entre 1/100 et 3/100, de préférence entre 1/100 et 2/100.

9. Procédé selon l'une quelconque des revendications précédentes **caractérisé en ce que** le tocophérol ou tocophérol protégé de formule (I) sous la forme de tocophérol ou tocophérol protégé de formule (I) pur ne comprend aucun triglycéride ni aucun acide gras.

10. Procédé de préparation d'un aliment ou d'une composition alimentaire avec une teneur réduite en déhydrotocophérol ou déhydrotocophérol protégé de formule (II) comprenant les étapes consistant à
a) préparer un tocophérol ou tocophérol protégé de formule (I) avec une teneur réduite en déhydrotocophérol ou déhydrotocophérol protégé de formule (II) selon le procédé des revendications 1 à 9 ; et
b) ajouter le tocophérol ou tocophérol protégé de formule (I) avec une teneur réduite en déhydrotocophérol ou déhydrotocophérol protégé de formule (II) issu de l'étape a) à au moins un complément alimentaire ou aliment ou ingrédient alimentaire.
